# NEUE EUROPÄISCHE PATENTSCHRIFT

(11) **EP 0 918 791 B3**
(45) Veröffentlichungstag dieser Patentschrift: **18.01.2012**
(45) Hinweis auf die Patenterteilung: 03.04.2002
(21) Anmeldenummer: 97937562.3
(22) Anmeldetag: 11.08.1997
(51) Int. Cl.: C07J 53/00

(54) **VERFAHREN ZUR HERSTELLUNG VON DROSPIRENON UND ZWISCHENPRODUKTE DAVON**
PROCESS FOR PRODUCING DROSPIRENONE AND INTERMEDIATES THEREOF
PROCEDE DE PREPARATION DE DROSPIRENONE ET LEUR INTERMEDIAIRES

(30) Priorität: 12.08.1996 DE 19633685
(43) Veröffentlichungstag der Anmeldung: 02.06.1999
(60) Teilanmeldung: 01250277.9
(73) Patentinhaber: Bayer Pharma Aktiengesellschaft, 13353 Berlin (DE)
(72) Erfinder: MOHR, Jörg-Thorsten, D-10555 Berlin (DE); NICKISCH, Klaus, D-12307 Berlin (DE)
(74) Vertreter: Bertucco, Alberto
(86) Internationale Anmeldenummer: PCT/EP1997/004342
(87) Internationale Veröffentlichungsnummer: WO 1998/006738

(56) Entgegenhaltungen:
- EP-A- 0 051 143
- EP-A- 0 075 189
- WO-A-90/14344
- D. BITTLER ET AL: "Synthesis of Spirorenone - A Novel Highly Active Aldosterone Antagonist" ANGEWANDTE CHEMIE. INTERNATIONAL EDITION., Bd. 21, Nr. 9, 1982, WEINHEIM DE, Seiten 696-697, XP002047531

## Beschreibung

Die Erfindung betrifft Verfahren zur Herstellung von Drospirenon (6β,7β; 15β,16β-Dimethylene-3-oxo-17a-pregn-4-ene-21,17-carbolactone, DRSP), sowie 6β,7β;15β,16β-Dimethylene-5β-hydroxy-3-oxo-17a-androstane-21,17-carbolactone (ZK 90965) als Zwischenprodukt des Verfahrens.

Drospirenon (6β,7β; 15β,16β-dimethylene-3-oxo-17a-pregn-4-ene-21,17-carbolactone, DRSP, INN) ist als steroidaler Wirkstoff seit längerem bekannt (DE 26 52 761 C2 und DE 30 22 337 A1) und die Herstellung der letzten 4 Schritte erfolgt im Eintopfverfahren; bei dem nach der Hydrierung von Dimethylenpropinol ZK 34506 keine der durchlaufenen Zwischenstufen Dimethylenpropanol und 5-β-OH-DRSP isoliert werden (siehe nachfolgendes Schema).

Eine analoge Synthese, jedoch unter Anwendung einer Pyridiniumdichromat - Oxidation ist aus dem Stand der Techik bekannt [Angew. Chemie, 21, 9, (1982), Seiten 696-697]. Ähnliche Synthesen zur Herstellung von steroidalen 17,21-Carbolactonen werden auch innerhalb von EP-A-0 075 189 und EP-A-0 051 143 beschrieben, jedoch unter Beteiligung von mikrobiologischen Reaktionen. Oxidationen unter Beteiligung von Rutheniumverbindungen werden aber nicht darin offenbart.

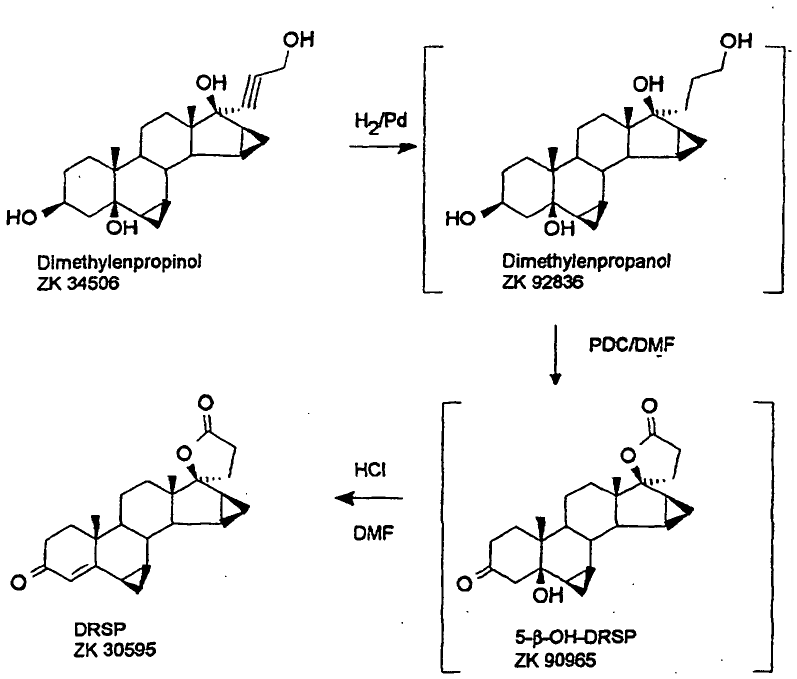

Das Dimethylenpropinol ZK 34506 wird in Tetrahydrofuran mit Wasserstoff an Palladium-Kohle zum Dimethylenpropanol ZK 92836 hydriert. Die so erhaltene Hydrierlösung, die das Propanol ZK 92836 als Hauptprodukt und schwankende Anteile an Lactol enthält, wird ohne Isolierung und Zwischenaufarbeitung zum Drospiroenon ZK 30595 (DRSP) umgesetzt.

Hierzu wird zuerst ein Lösungsmittelwechsel von Tetrahydrofuran zu Dimethylformamid vollzogen und anschließend das Propanol bei 40° C mit einem Überschuß von 3,7 Equivalenten Pyridiniumdichromat (PDC) zu einem Gemisch von DRSP und 5-β-OH-DRSP oxidiert. Die 5-β-OH-Funktion im Oxidationsprodukt ist labil gegenüber Säuren, Lewissäuren und basischen Bedingungen bei erhöhten Temperaturen, da in allen Fällen mit der Ausbildung des Δ-4,5-ungesättigten Ketons im Drospirenon ein thermodynamisch stabileres Produkt erhalten wird. Die Eliminierung der β-OH-Funktion im 5-β-OH-DRSP verläuft zum thermodynamisch stabileren Drospirenon und konnte nicht unterdrückt werden konnte.

Die Mischung enthält in der Regel wechselnde Anteile der beiden Komponenten, wobei das 5-β-OH-DRSP im allgemeinen als Hauptkomponente im Verhältnis von 2-3:1 vorliegt. In der letzten Stufe der Eintopfsequenz wird das Zweikomponenten-Gemisch durch Zugabe von halbkonzentrierter Salzsäure in das DRSP, roh überführt.

In der nachstehenden Tabelle sind die letzten vier Betriebsansätze zusammengefaßt.

| Ansatz | Ausbeute, roh (%) | Reinheit (100%-Methode) |
|---|---|---|
| 537201 | 57,2 | 98,9 |
| 202 | 63,7 | 99,09 |
| 203 | 46,5 | 99,18 |
| 204 | 58,3 | 98,81 |
| **Gesamt** | **mittlere Ausbeute: 56,4** | **mittlere Reinheit: 98,9** |

Im Mittel aller Betriebsansätze wird ausgehend vom Dimethylenpropinol eine theoretische Ausbeute von 56% DRSP, roh in einer HPLC-Reinheit von 98,9% erzielt.

Aufgabe der Erfindung ist die Bereitstellung eines neuen Herstellungsverfahrens für Drospirenon, welches selektiver und einfacher in der Durchführung ist, als jenes aus dem Stand der Technik und außerdem ökologischer ist; (Einsparung einer Chromtrioxid-Oxidation).

Gelöst wird diese Aufgabe gemäß der Lehre der Ansprüche.

Die Erfindung beinhaltet ein Verfahren zur Herstellung von Drospirenon (6β,7β; 15β,16β-dimethylene-3-oxo-17a-pregn-4-ene-21,17-carbolactone, DRSP) 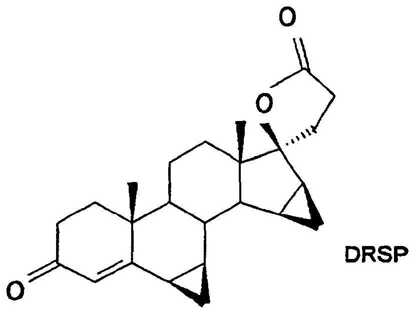 durch katalytische Hydrierung von 17α-(3-hydroxy-1-propynyl)-6β,7β;15β,16β-dimethylene-5β-androstane-3β,5,17β-tnol (ZK 34506) 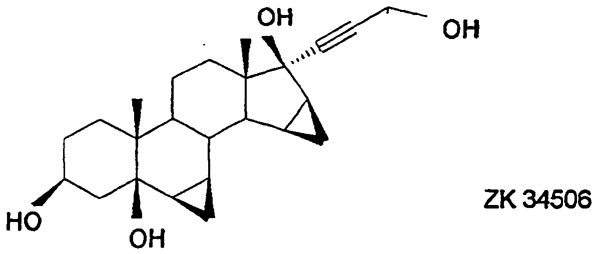 in das 17α-(3-hydroxy-1-propyl)-6β,7β; 15β,16β-dimethylene-5β-androstane-3β,5,17β-triol (ZK 92836) 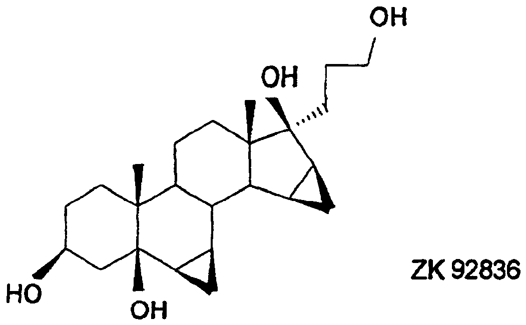 anschließende Oxidation mittels käuflicher Rutheniumsalze wie RuCl₃, RuO₂, KRuO₄, K₂RuO₄, vorzugsweise jedoch in Gegenwart katalytischer Mengen von RuCl₃ (lmol%), und herkömmlichen, einfachen Oxidationsmitteln wie ^{t}Butylhydroperoxyd, N-Methyl-morpholin-N-oxid, M₂S₂O₈ (M=Na, K), MXOy (M=Li, Na, K ; X=B, Cl, Br, I; y=1-4), vorzugsweise jedoch 1-3 Equivalenten NaBrO₃, in Lösungsmitteln wie Acetonitril, Chloroform, Methylenchlorid, Tetrachlorkohlenstoff, Wasser, Tetrahydrofuran, tert-Butanol, Ethylacetat oder Kombinationen hiervon, vorzugsweise jedoch in einem Acetonitril-Wasser-Gemischin der Zusammensetzung Acetonitril: Wasser = 1:1, in das 6β,7β;15β,16β-dimethylene-5β-hydroxy-3-oxo-17α-androstane-21,17-carbolactone (ZK 90965) 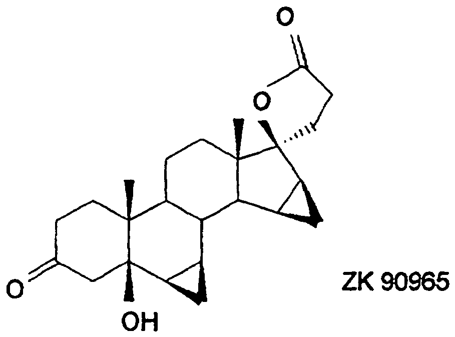 und anschließende Wasserabspaltung.

Die Erfindung beinhaltet als eine Schlüssel reaktion die Ruthenium katalysierte Oxidation von Dimethylenpropanol ZK 92836 zum 5-(3-OH-DRSP ZK 90965 und die anschließende Wassereliminierung zum Drospirenon ZK 30595 in einem zweistufigen Verfahren. Solche Oxidationen werden beispielsweise innerhalb der WO-A-90/14344 an aliphatischen gesättigten α, ω-Diolen zu den entsprechenden Lactonen beschrieben.

Analog zum bekannten Verfahren aus dem Stand der Technik wird im erfindungsgemäßen Verfahren Dimethylenpropinol ZK 34506 in Tetrahydrofuran mit Wasserstoff an Palladium-Kohle hydriert. Die Hydrierlösung wird anschließend einem Lösungsmittelwechsel von Tetrahydrofuran auf Acetonitril unterworfen. Die Acetonitril-Lösung wird mit einer katalytischen Menge Rutheniumtrichlorid (1 mol%) und 3 Equivalenten Natriumbromat bei 40°-60° C gezielt zum 5-β-OH-DRSP oxidiert. Trotz der großen Labilität des 5-β-OH-DRSP gegenüber Säuren, Lewissäuren wie beispielsweise der Chromverbindungen im alten Betriebsverfahren, starken Basen oder hohen Temperaturen, die in allen Fällen auf die hohe Triebkraft zur Bildung des thermodynamisch stabileren Δ-4,5-ungesättigten Ketons zurückzuführen ist, gelingt unter den gewählten Reaktionsbedingungen die selektive Synthese des 5-(3-OH-DRSP, ohne das eine Drospiroenonbildung zu beobachten ist. Das 5-β-OH-DRSP kann durch eine (betrieblich) einfach durchzuführende Wasserfällung aus der Reaktionslösung isoliert werden.

Die Ausbeuten liegen im Bereich von 68% bis 75% über die beiden Stufen Hydrieren und anschließende Oxidation.

Aus eigenen Versuchen ist bekannt, daß das Drospiroenon bei Säureeinwirkung nach zwei Reaktionswegen zersetzt werden kann. Einmal wird das Drospiroenon unter sauren Bedingungen leicht in das epimere Isolacton ZK 35096 überführt.

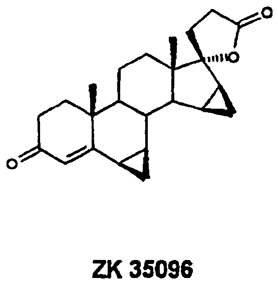

Das zweite Nebenprodukt entsteht durch einen HCI-Angriff auf die 6,7-Methylengruppe, der zu dem Ringöffnungsprodukt ZK 95673 führt.

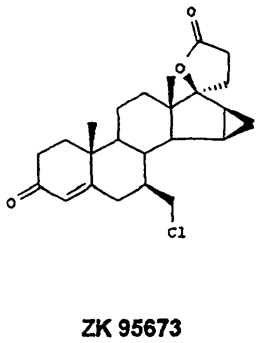

Beide Nebenprodukte werden unter den Reaktionsbedingungen des neuen Verfahrens soweit zurückgedrängt, daß sie nur noch in einer Größenordnung von <0,2% zu beobachten sind.

Bei der Eliminierung wird eine Ausbeute von 96% d.Th. erzielt. Die Gesamtausbeute des neuen Verfahrens liegt damit im Bereich von 65% bis 72% d. Theorie.

Ein weiterer sehr wesentlicher Vorteil des erfindungsgemäßen Verfahrens gegenüber dem Stand der Technik liegt im Bereich der Ökologie. Es ist gelungen, die bisher verwendeten toxischen Chromverbindungen, die in Form der Pyridiniumdichromat -Salze bislang zur Oxidation verwendet wurden und hinterher in Form ihrer Lösungen entsorgt werden müssen, durch katalytische Mengen eines Metalls zu ersetzen. Zudem ist es möglich, daß eingesetzte Acetonitril-Wasser-Gemisch durch azeotrope Destillation zu recyclen, so daß auch keine Gefahrfürdie Umwelt zu erwarten ist.

Weiter beinhaltet die Erfindung das Zwischenprodukt 6β,7β;15β,16β-Dimethylene-5β-hydroxy-3-oxo-17α-androstane-21,17-carbolactone (90965).

### Beispiele:

### 6β,7β;15β,16β-dimethylene-5β-hydroxy-3-oxo-17α-androstane-21,17-carbolactone

50 g 17α-(3-Hydroxy-1-propynyl)-6β,7β; 15β,16β-dimethylene-5β-androstane-3β,5,17β-triol wird in 1000 ml THF in der Gegenwart von 10 g Palladium auf Kohle (10%) und 3 ml Pyridin hydriert bis 2 Equivalente Wasserstoff aufgenommen werden. Anschließend wird der Katalysator abfiltriert und die Lösung bis zur Trockne eingeengt, wobei 52,7 g 7a-(3-hydroxy-1-propyl)-6β,7β; 15β,16β-dimethylene-5β-androstane-3β,5,17β-triol erhalten werden, die ohne Reinigung weiter umgesetzt werden.

50, 2 g 7α-(3-hydroxy-1-propyl)-6β,7β; 15β,16β-dimethylene-5β-androstane-3β,5,17β-triol werden in 250 ml Acetonitril suspendiert und auf 45° C erwärmt. Hierzu werden 0,52g Rutheniumtrichlorid gelöst in 10 ml Wasser und 62,46 g Natriumbromat gelöst in 250 ml Wasser getropft. Es wird 2 Stunden bei 50° C nachgerührt und die Lösung anschließend durch Zugabe von 1000 ml Wasser gequencht. Es werden 200 ml Ethylacetat zugesetzt, die Phasen getrennt und anschließend die wäßrige Phase mit 600 ml Ethylacetat extrahiert. Die vereinigten organischen Phasen werden über Natriumsulfat getrocknet und anschließend bis zur Trockne eingeengt. Hierbei werden 43,44 g 6β,7β; 15β,16β-Dimethylene-5β-hydroxy-3-oxo-17α-androstane-21,17-carbolactone als Rohprodukt erhalten. Durch Umkristallisieren aus Aceton-Isoether erhält man 35,7g 6β,7β;15β,16β-Dimethylene-5β-hydroxy-3-oxo-17α-androstane-21,17-carbolactone mit einem Schmelzpunkt von 216°-218°C. Der Drehwert liegt bei -65,6° (Natriumlinie, c=1,02 in CHCl3).

### 6β,7β; 15β,16β-Dimethylene-3-oxo-17α-pregn-4-ene-21,17-carbolactone

28 g 6β,7β;15β,16β-dimethylene-5β-hydroxy-3-oxo-17a-androstane-21,17-carbolactone werden in 280 ml THF suspendiert und anschließend mit 10 mol%, 1,5g p-Toluolsulfonsäure versetzt Nach 30 Minuten werden 125 ml ges. NaCl-Lösung und 8,2 ml 1 n NaOH-Lösung zugegeben. Nach Phasentrennung wird die organische Phase über Natriumsulfat getrocknet und bis zur Trockne eingengt, wobei 25,67g 6β,7β; 15β,16β-dimethylene-3-oxo-17a-pregn-4-ene-21,17-carbolactone als Rohprodukt erhalten, dessen Reinheit nach HPLC-Bestimmung bei 93% liegt.

Die weitere Aufreinigung kann durch Chromatographie erfolgen.

Der Schmelzpunkt der chromatographierten Substanz liegt bei 197,5° -200° C.

## Patentansprüche

1. Verfahren zur Herstellung von Drospirenon (6β,7β; 15β,16β-dimethylene-3-oxo-17α-pregn-4-ene-21,17-carbolactone, **DRSP**) 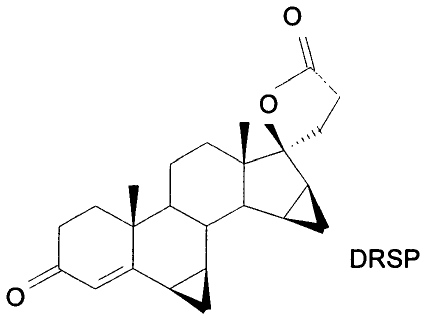 durch katalytische Hydrierung von 17α-(3-hydroxy-1-propynyl)-6β,7β;15β,16β-dimethylene-5β-androstane-3β,5,17β-triol (**ZK 34506**) 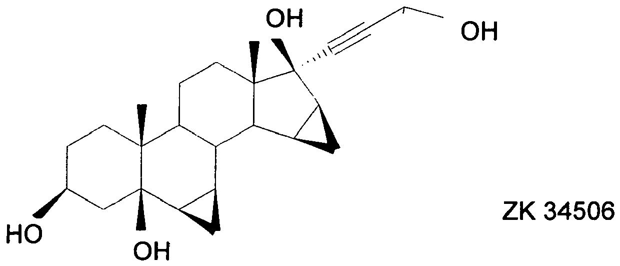 in das 7α-(3-hydroxy-1-propyl)-6β,7β; 15β,16β-dimethylene-5β-androstane-3β,5,17β-triol (**ZK 92836**) 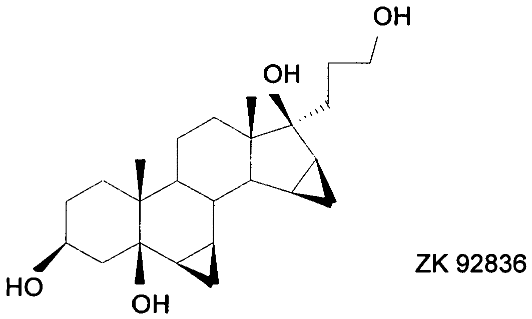 anschließende Oxidation in Gegenwart eines Rutheniumsalzes in das 6β,7β;15β,16β -dimethylene-5β-hydroxy-3-oxo-17α-androstane-21,17-carbolactone (**ZK 90965**) 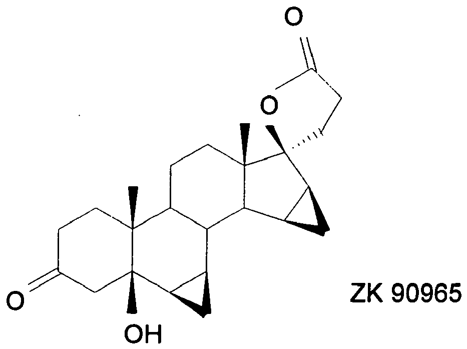 und anschließende Wasserabspaltung.

2. 6β,7β;15β,16β-Dimethylene-5β-hydroxy-3-oxo-17α-androstane-21,17-carbolactone (**ZK 90965**)

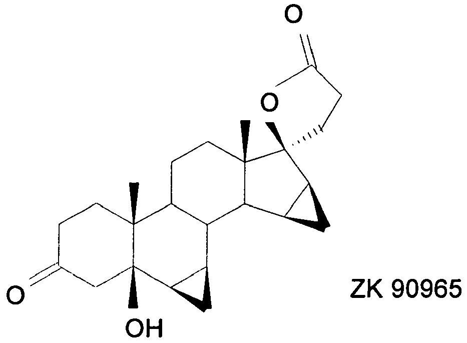

3. Verfahren zur Herstellung von Drospirenon nach Anspruch 1, **dadurch gekennzeichnet, daß** im erhaltenen Produkt weniger als 0,2% Verunreinigung an dem Isolacton ZK 35096 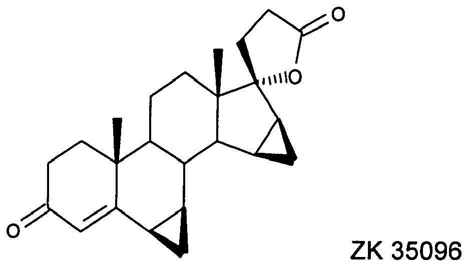 und dem durch sauren Angriff auf die 6,7-Methylengruppe entstandenen 6,7-Ringöffnungsprodukt enthalten sind.

4. Verfahren zur Herstellung von Drospirenon, das weniger als 0,2% Verunreinigung an dem lsolacton ZK 35096 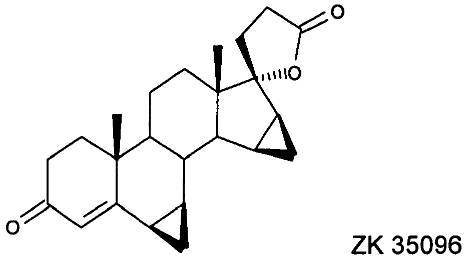 und dem durch sauren Angriff auf die 6,7-Methylengruppe entstandenen 6,7-Ringöffnungsprodukt enthält umfassend Wasserabspaltung aus 6β,7β,15β,16β-dimethylene-5β-hydroxy-3-oxo-17a-androstane-21,17-carbolactone (ZK 90965) durch Zugabe von 10 mol % p-Toluolsulfonsäure.

## Claims

1. A process for the production of drospirenone (6β,7β;7β,16β-dimethylene-3-oxo-17α-pregn-4-ene-21,17-carbolactone, **DRSP**) 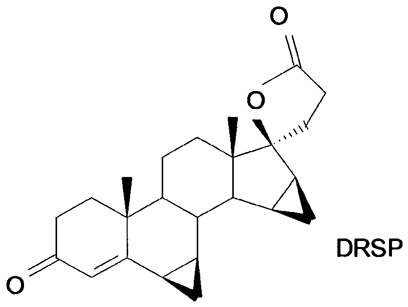 comprising, catalytically hydrogenating 17α-(3-hydroxy-1-propynyl)-6β,7β;15β,16β-dimethylene-5β-androstane-3β,5,17β-triol (**ZK 34506**) 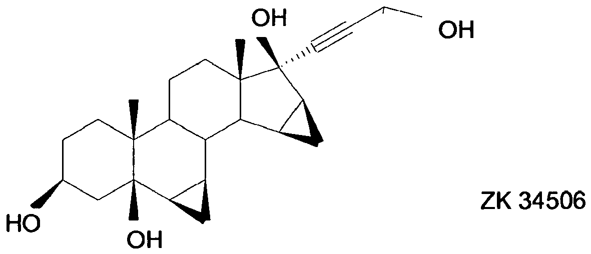 into 7α-(3-hydroxy-1-propyl)-6β,7β; 15β,16β-dimethylene-5β-androstane-3β,5,17β-triol (**ZK 92836**) 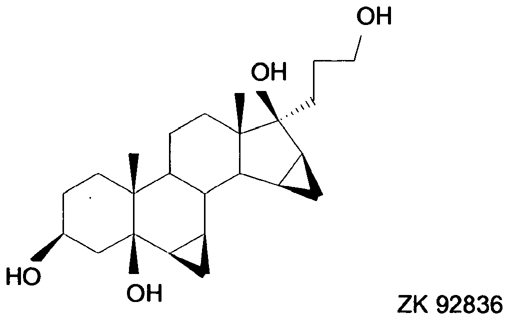 oxidising the latter in the presence of ruthenium salt to form 6β,7β;15β,16β-dimethylene-5β-hydroxy-3-oxo-17α-androstane-21,17-carbolactone (**ZK 90965**) 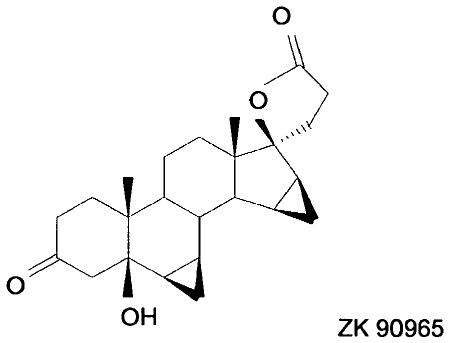 and dehydrating the latter.

2. 6β,7β;15β,16β-Dimethylene-5β-hydroxy-3-oxo-17α-androstane-21,17-carbolactone (**ZK 90965**)

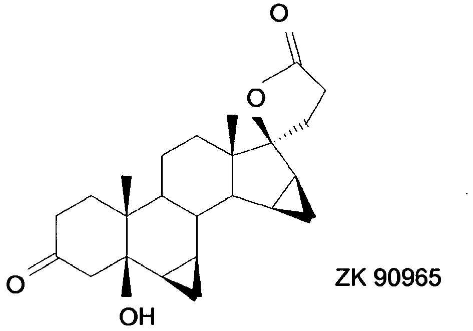

3. A process for the production of drospirenone according to claim 1, **characterised in that** the resulting product comprises less than 0,2% 01 the contaminants isolactone ZK 35096 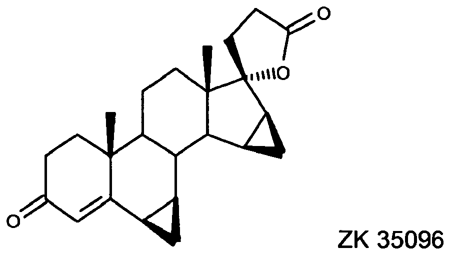 and the ring-opened product formed by the acid-catalysed ring-opening reaction of the 6,7-methylene group.

4. A process for the production of drospirenone, comprising less than 0,2% of the contaminants isolactone ZK 35096 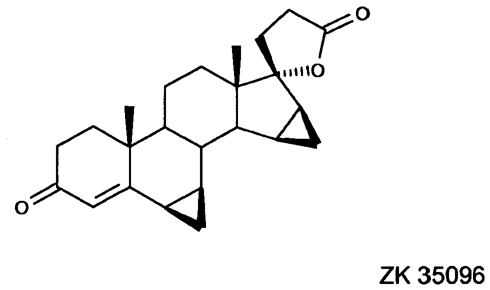 and the ring-opened product formed by the acid-catalysed ring-opening reaction of the 6,7-methylene group, comprising water elimination from 6β,7β;15β,16β-dimethylene-5β-hydroxy-3-oxo-17α-androstane-21,17-carbolactone (ZK 90965) by addition of 10 mol % p-toluenesulfonic acid.

## Revendications

1. Procédé de préparation de la drospirénone (6β,7β;15β,16β- diméthylène -3-oxo-176β,7β;15β,16β-pregn-4-ene-21,17-carbotactone, **DRSP**) 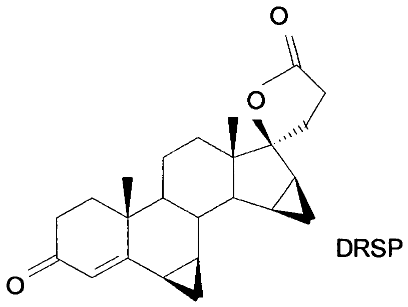 par hydrogénation catalytique du 17α-(3-hydroxy-1-propynyl)-6β,7β;15β,16β-diméthylène -5β-androstane-3β,5,17β-triol (**ZK 34506**) 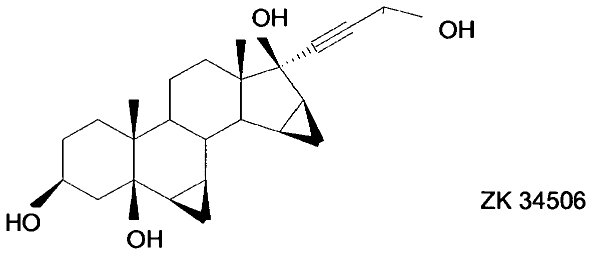 en 7-(3-hydroxy-1-propyl)-6β,7β; 15β,16β-diméthylène-5β-androstane-3β,5,17β-triol (**ZK 92836**), 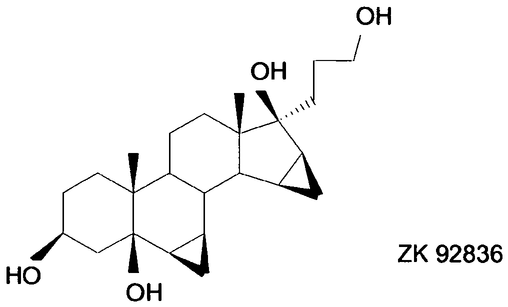 par oxydation en présence d'un sel de ruthénium dans la 6β,7β;15β,16β-diméthylène-5β-hydroxy-3-oxo-17α-androstane-21,17-carbolactone (**ZK 90965**) 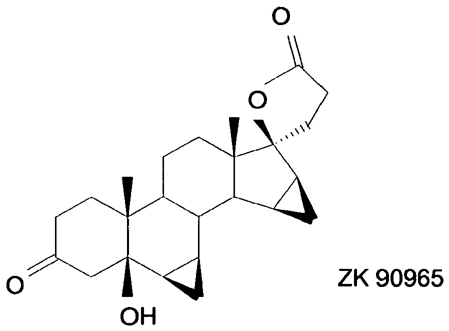 et par clivage subséquent d'eau.

2. 6β,7β;15β,16β- diméthylène -5β-hydroxy-3-oxo-17α-androstane-21,17-carbolactone (**ZK 90965**)

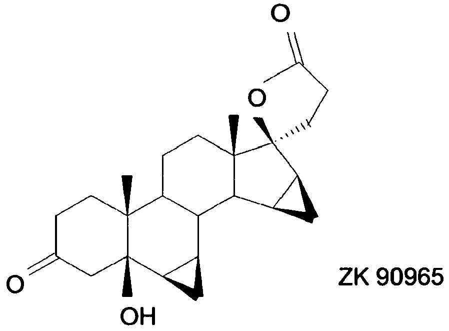

3. Procédé de préparation de la drospirénone selon la revendication 1, **caractérisé en ce que**, dans le produit obtenu, sont contenus moins de 0,2 % d'impuretés en provenance de l'isolactone ZK 35096 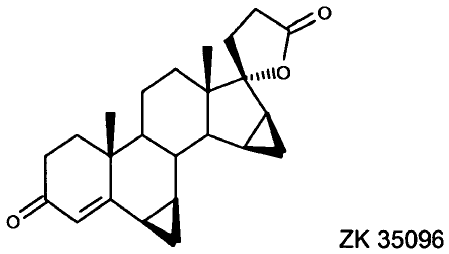 et du produit d'ouverture du cycle 6,7 se formant par attaque acide sur le groupement 6,7-méthylène.

4. Procédé de préparation de la drospirénone contenant moins de 0,2 % d'impuretés en provenance de l'isolactone ZK 35096 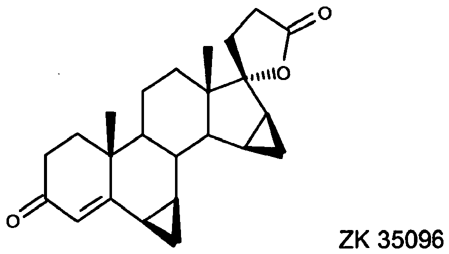 et du produit d'ouverture du cycle 6,7 se formant par attaque acide sur le groupement 6,7-méthylène, comprenant le clivage d'eau à partir de la 6β,7β,15β,16β-dimethylene-5β-hydroxy-3-oxo-17α-androstane-21,17-carbolactone (ZK 90965) par addition de 10 mol% de l'acide p-toluènesulfonique.
